(19)

Europäisches Patentamt

European Patent Office

Office européen des brevets

(11) **EP 3 424 321 A1**

(12) **EUROPEAN PATENT APPLICATION**

(43) Date of publication:
09.01.2019 Bulletin 2019/02

(51) Int Cl.:
*A01N 27/00* [(2006.01)]   *A01P 7/00* [(2006.01)]
*A61K 31/01* [(2006.01)]

(21) Application number: 17382439.2

(22) Date of filing: 06.07.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Ferrer Internacional, S.A.
08028 Barcelona (ES)

(72) Inventors:
• **Bacchini, Gabriela Silvina**
  **08029 Barcelona (ES)**
• **Puig Algora, Gemma**
  **08950 Esplugues de Llobregat (ES)**

(74) Representative: **Ferrer Internacional S.A.**
**Joan Buscallà 1-9**
**08173 Sant Cugat del Vallès (ES)**

(54) **COMPOUNDS FOR TREATING ECTOPARASITE INFESTATION**

(57)    The present invention relates to compounds for killing ectoparasites, in particular head lice and body lice as well as their eggs.

EP 3 424 321 A1

**Description**

The present invention relates to compounds for killing ectoparasites and/or their ova on a subject, methods and compositions thereof.

**BACKGROUND OF THE INVENTION**

Ectoparasites are organisms which inhabit the skin or outgrowths of the skin of another organism (the host) for various periods, and may be detrimental to the latter. Various ectoparasites cause significant infestations in humans and in many kinds of domestic animals including livestock, pets, laboratory animals, poultry, fish and bees. Many of these ectoparasites (e.g. most lice) are host specific, while others (e.g. many ticks) parasitize a wider range of hosts. Many ectoparasites are known to be vectors of pathogens, which the parasites typically transmit to hosts while feeding or (occasionally) defecating. Examples of ectoparasites are lice, ticks, mites and fleas.

Lice are external parasites of warm blooded animals. Humans are host to three different types of lice: headlice, body lice, and crabs or pubic lice.

Head lice infestation (*pediculosis capitis*) is a persistent problem with as many as 6-12 million people worldwide affected each year. The problem is particularly prevalent in children with preschool and elementary-age children aged 3-10 and their families becoming infested most often.

Head lice infestation is produced by the common head louse *Pediculus humanus capitis*, and typically causes itching of the scalp. As the lice feed on human blood, they may cause lesions to develop on the scalp, swollen glands on the neck or under arms, or other symptoms. Head lice infestation causes serious problems due to the negative social implications of the infestation. Body lice (*Pediculus humanus corporis*) are also bothersome to humans and carry the additional hazard of being the vectors of certain diseases, such as exanthematic or epidemic typhus and recurrent fever.

The life cycle of the head louse falls into three phases: egg, nymph and mature louse.

The louse's hard chitinous exoskeleton serves as protection from external elements. Lice eggs (or ova) are similarly protected by a chitinous sheath surrounding the eggs and attached to the hair shaft. In the case of lice these ova are also referred to as nits. Most eggs take 7 days to hatch (but a few may take longer, up to 13 days) and may appear visible for weeks after death of the egg. Although lice may be affected by the use of an insecticide, the eggs often remain resistant to attack. Thus, optimum treatment of a lice infestation includes both a pediculicide, which kills the adult lice and nymphs, and an ovicide, which interrupts the development of the eggs.

Treatment options for head lice infestation can broadly be divided into five groups as follows: topically applied insecticides; topically applied physically acting agents; topically applied homeopathic, plant formulations and other remedies; oral drugs; mechanical agents (combs, electronic devices, heating devices). Various of these compositions are available for treating lice infestations, which generally take a topical approach to treatment. Most of these treatments involve the use of insecticides that are harsh agents, thus raising toxicity concerns. The lice can also become resistant to the insecticides used and therefore the compositions can lose their effectiveness over time.

Eradication of head lice involves total removal or destruction of the mature lice, nymphs and the eggs on each host. Various attempts have been proposed to achieve such destruction but none are wholly satisfactory.

US 4,147,800 A discloses that aliphatic carbocyclic esters exhibit pediculicidal properties if used at concentrations above 70 % of the composition and teaches the use of these esters in combination with aliphatic alcohols.

US 6,303,581 teaches a variety of surfactants, including non-volatile lipids, non-volatile fatty alcohols and non-volatile fatty esters or mixtures thereof, that are water-soluble or aqueous-based and have been found to be pediculostatic.

GB 2204243B2 discloses a method for controlling lice or their ova on human subjects consisting of applying a topical composition comprising a lousicide and at least a C2-C4 alkyl ester of a C10-C20 fatty acid dissolved in an alcoholic solution. Preferably the lousicide comprises carbaryl, malathion or phenothrin.

WO00/01347 discloses compositions containing spinosad, siloxanes and fatty acid esters.

WO2001019190 discloses compositions that contain 85 to 99.9 % cyclosiloxanes as pediculicides.

WO2001040446 discloses the ectoparasicidal activity of a composition comprising isopropylmyristate, octyl palmitate and ivermectin.

WO03092583 discloses compositions for killing ectoparasites on a subject containing a fatty acid ester, e.g., isopropyl myristate, effective for killing ectoparasites. Also described are compositions containing a fatty acid ester and a siloxane (e.g. decacyclomethicone).

US2013/0018016A1 discloses a composition for killing ectoparasites and/or their eggs, comprising at least one volatile at room temperature, liquid, non-polar organic solvent, 1 to 10 wt.-%, based on the total composition, of at least one spreading agent and 35 to 65 wt.-%, based on the total composition, of at least one polysiloxane having a viscosity of greater than 90 cSt.

The need for further treatment increases the exposure to these harsh agents and increases the cost. Additionally, clinicians and parents are reluctant to treat children with agents that can also prove toxic to human beings. There are reports in the medical literature, for example, that children treated with lindane have developed seizures.

Moreover, many of these compounds have unpleasant odours or other undesirable properties, causing non-compliance by the patient, leading to re-infestation of the individual, and spreading of the infestation to others. In addition, the harshness of these agents make them unsuitable for use as prophylactics.

Thus, there remains a need in the art for methods and kits useful for treating and removing ectoparasites infestations, such as lice infestations, that are easy to use, cosmetically attractive and effective against ectoparasites resistant to other treatments. Moreover, there remains a need for methods and kits that eradicate nits, also referred to as eggs or ova, as well as adults and instars, and that can be used prophylactically to prevent initial infection or re-infestation. Accordingly, these are some of the objects of the present invention.

Thus, the present invention has been made in view of the above problems and it is an object of the invention to provide a safe and effective method of eradicating ectoparasites, especially lice. In particular, to provide an effective and pediculicidal composition for treatment of lice infestations, more specifically head lice infestations. It is a further object of this invention to provide a compound or composition which will be effective in the prevention of head lice infestation.

The above issues have been addressed in the present invention and specific embodiments thereof.

## DESCRIPTION OF THE INVENTION

The present invention provides squalane for use as a medicament. More specifically, squalane for use in killing ectoparasites and/or their ova on a subject. It also provides compositions and methods for killing ectoparasites and/or their ova on a subject. In the most preferred embodiments, the subject is a human and the ectoparasites are lice, fleas, mites and ticks.

The inventors discovered unexpectedly that the compound squalane is particularly effective in killing ectoparasites on a subject as well as their ova. The present inventors also discovered that the combination of squalane and silicones (e.g., a cyclomethicone) can also kill ectoparasites. Thus, squalane alone or squalane in combination with silicones may be included in a composition in an amount effective for killing lice, fleas, mites, ticks and other ectoparasites to result in a composition that is effective for this purpose.

In a first aspect of the first invention, it is provided squalane for use as a medicament.

In a further embodiment of the first aspect, squalane is for use in killing ectoparasites and/or their ova on a subject. Squalane is effective in killing ectoparasites as well as their ova, meaning one or the other or both.

Squalane is frequently used in cosmetics as emollient and moisturizer. However, there is no indication in the prior-art that has any effect on ectoparasites. Squalane has low acute toxicity and is not an irritant at the concentrations used in cosmetics (up to 100%). Squalane, a saturated aliphatic hydrocarbon ($C_{30}H_{62}$), is obtained by hydrogenation of squalene, which has been used in cosmetics as a moisturizer. Squalene, a precursor for squalane, is contained in shark liver oil, rice, olive, soybean, and so on. Squalane (2,6,10,15,19,23-hexamethyltetracosane) is a clear, odourless and colourless oil of very low polarity. It has a density (20 °C) of 0.800 - 0.815 g/cm$^3$.

According to this invention by the term "ectoparasite" it is meant a parasite that lives outside the body of the host, on the skin or outgrowths of the skin of the host.

In a preferred embodiment, the ectoparasites are non-flying ectoparasites. In another preferred embodiment, the ectoparasites are selected from the group consisting of lice, mites, ticks and fleas.

In another preferred embodiment, the subject is a mammal or plant, preferably the subject is a mammal. In another preferred embodiment, the mammal is a human. In the most preferred embodiment the ectoparasites are head lice.

The term "subject" includes plants and mammals. The term "mammal" includes humans, and also includes animals that are members of the class Mammalia. This will usually be a human but also includes pets such as dogs, cats, ferrets, rabbits, gerbils, and guinea pigs. Mammals also include domestic animals such as bovines, porcines, ovines, and equines. While most fur-bearing animals can become infested with fleas and ticks, pigs, horses, and cattle can also be infested with lice (e.g., the Haematopinus suis, which infests pigs, and other Haematopinus spp. that infest horses and cattle). These infestations are treatable with the product and compositions described herein.

By "infestation" is meant the presence of lice, fleas, ticks or other ectoparasites that are the target of the treatment. Ectoparasites or pests include, but are not limited to, head lice, body lice (e.g., Pediculus humanus), crab lice (e.g., Phthirus pubis), mites (scabies), fleas and ticks. The presence of eggs of the target ectoparasite also constitutes infestation. Thus, optimum treatment of an ectoparasites infestation includes both the erradication of adult parasites and their eggs (ova). In the case of lice infestation, it includes both a lousicidal effect, which kills the adult lice and nymphs, and an ovicidal effect, which interrupts the development of the eggs. In a preferred embodiment, the ectoparasite egg or ova is a louse egg, more preferably head louse egg.

In a second aspect of the first invention, it is provided a composition for use in killing ectoparasites and/or their ova on a subject comprising squalane, wherein squalane is present in an amount of at least 5 % by weight in respect of the total amount of the composition.

The term "% by weight" in the present invention refers to **% w/w** (weight per weight) in respect of the total amount of the composition.

The compositions as herein disclosed offer the surprising and highly desirable combination of benefits of being highly effective, spreading evenly, drying quickly, having low mammalian toxicity, and being hair and skin compatible by not having a greasy or oily texture. Therefore, the compositions of the present invention eliminate the disadvantages of previously available compositions of being messy and inconvenient to apply, emitting an unpleasant odour, having limited effectiveness, or having substantial mammalian toxicity.

The compositions do not require the presence of any alcohol since the treated patient will have bites and lesions on the scalp or body caused by the ectoparasites, and the application of compositions containing alcohols will cause pain and discomfort. Thus, the compositions do not need to contain aliphatic alcohols or any other alcohols. The compositions as herein disclosed have shown ovicidal (kill eggs/ova), lousicidal (kill lice), or pediculicidal (kill both eggs and lice) activity or efficacy.

In a further embodiment of the second aspect, the composition for use does not comprise Lippia javanica essential oil. As used herein, "Lippia javanica oil" refers to the oil from a species of the family Verbenaceae, which is a family of herbs and shrubs or small trees that have aromatic leaves. It includes oil from the species Lippia javanica (Burm f.) Spreng. It is known by its common name, fever tree. Lippia javanica oil is also known as zinziba oil. Lippia javanica grows in South Africa, Swaziland, Zambia, Botswana, Kenya, Malawi, Tanzania and Mozambique.

In a preferred embodiment of the composition as herein disclosed, the total amount of squalane present in the composition ranges from 5 to 20 % by weight in respect of the total amount of the composition. Preferably, squalane is present in an amount of more than 20 % by weight in respect of the total amount of the composition.

In a preferred embodiment of the composition as herein disclosed, squalane is present in an amount of at least 30% by weight in respect of the total amount of the composition, preferably in an amount of at least 40 % by weight in respect of the total amount of the composition, more preferably in an amount of at least 50 % by weight in respect of the total amount of the composition, even more preferably in an amount of at least 60 % by weight in respect of the total amount of the composition.

In another preferred embodiment of the composition as herein disclosed, the ectoparasites are selected from the group consisting of lice, mites, ticks and fleas. In another preferred embodiment, the subject is a mammal or plant, preferably the subject is a mammal. In another preferred embodiment, the mammal is a human. In the most preferred embodiment the ectoparasites are head lice.

In a further embodiment of the composition as herein disclosed, said composition further comprises at least a siloxane. Said siloxane is preferably a cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane.

Siloxanes are useful in the present invention. A siloxane is a volatile, cyclic, non-polymeric silicone that dries quickly, spreads evenly, and does not leave a greasy residue. Cyclomethicones are a group of methyl siloxanes, a class of liquid silicones (cyclic polydimethylsiloxane polymers) that possess the characteristics of low viscosity and high volatility as well as being skin emollients and in certain circumstances useful cleaning solvents.

The USP32-NF27 describes cyclomethicone as a fully methylated cyclic siloxane containing repeating units of the formula $[-(CH_3)_2SiO-]_n$ in which n is 4, 5, or 6, or a mixture of them. The cyclomethicone used in the present invention is clear, colourless, volatile carrier that provides a soft silky feel. It is composed preferably mainly of decamethylcyclopentasiloxane (D5), which is present at greater than 97%. The octamethylcyclotetrasiloxane (D4) is present at values less than 0.9% in cyclomethicone.

In a preferred embodiment of the composition as herein disclosed, the total amount of siloxane or siloxanes present in the composition ranges from 30 to 70 % by weight in respect of the total amount of the composition, preferably ranges from 35 to 65 % by weight in respect of the total amount of the composition, more preferably ranges from 40 to 55 % by weight in respect of the total amount of the composition.

In a further embodiment of the composition as herein disclosed, said composition comprises at least a further emollient. Said further emollient is preferably a fatty acid ester, more preferably selected from cetyl palmitate, decyl oleate, ethyl oleate, isopropyl myristate, isopropyl palmitate, ethylhexyl stearate and mixtures thereof, even more preferably the composition comprises ethylhexyl stearate (i.e. 2-ethylhexyl stearate). Ethylhexyl stearate is colourless to slightly yellow liquid ester with medium spreading properties. It has a density approx. of 0.85 $g/cm^3$.

By "fatty acid ester" is meant a type of ester that result from the combination of a fatty acid with an alcohol, e.g. isopropyl myristate. "Fatty acid" refers to a carboxylic acid with a long aliphatic chain, which is either saturated or unsaturated. Most naturally occurring fatty acids have an unbranched chain of an even number of carbon atoms, from 4 to 28. An ester is a functional derivative of a carboxylic acid, where the -OH group of the carboxylic acid has been replaced by an -OR, R being an alkyl group.

In a preferred embodiment of the composition as herein disclosed, the total amount of fatty acid ester or fatty acid esters present in the composition ranges from 1 to 10 % by weight in respect of the total amount of the composition, preferably ranges from 3 to 7 % by weight in respect of the total amount of the composition.

In a preferred embodiment of the composition as herein disclosed, the total amount of squalane present in the composition is present in an amount of more than 20 % by weight in respect of the total amount of the composition, the ectoparasites are head lice, the composition comprises decamethylcyclopentasiloxane in an amount that ranges from 35 to 65 % by weight in respect of the total amount of the composition and the composition comprises ethylhexyl stearate in an amount that ranges from 3 to 7 % by weight in respect of the total amount of the composition.

In a further embodiment of the composition as herein disclosed, said composition further comprises tea tree oil. In a preferred embodiment, the total amount of tea tree oil present in the composition ranges from 0.5 to 5% % by weight in respect of the total amount of the composition, preferably ranges from 1 to 3 % by weight in respect of the total amount of the composition. As used herein, tea tree oil refers to Tea Tree (Melaleuca alternifolia) oil. "Melaleuca alternifolia oil" refers to the essential oil from the bottlebrush tree and is commonly called tea tree oil. Melaleuca alternifolia is indigenous to New Zealand and Australia but also grows in Southern California. The oil usually is produced by distillation of the leaves. Melaleuca alternifolia oil has a medicinal odour although some people characterize the smell as a pungent spicy woodsy scent. The chemical constituents of Melaleuca alternifolia oil include various levels of [alpha]-pinene, sabinene, [alpha]- terpinene, limonene, p-cymene, 1,8-cineole, [gamma]-terpinene, terpinolene, terpinen-4-ol, [alpha]-terpineol, aromadendrene, d-cadinene, globulol, and viridiflorol. The oil is commercially available (e.g., see J. Rose, The Aromatherapy Book - Applications & Inhalations (North Atlantic Books, 1992); and from Berje Essential Oils, Bloomfield, NJ; Liberty Natural Products, Portland, OR; and Mountain Rose Herbs, Eugene, OR).

In a further embodiment of the composition as herein disclosed, the composition consists only of squalane.

The compositions of the invention are intended to expose the ectoparasites for a period of time to them. After a given time of this exposure ectoparasites and/or their ova are killed. The compositions of the invention are formulated to be applied on the zone infested by ectoparasites.

Preferably, the compositions are formulated to be applied to the scalp of a subject suffering from a head lice infestation and are left on the treated subject for a period of time. In a further embodiment of the composition as herein disclosed, at least 75% of ectoparasites present are killed within 4 hours after a 10 minute exposure to the composition, preferably at least 75% of ectoparasites present are killed within 2 hours after a 10 minute exposure to the composition, preferably at least 75% of ectoparasites present are killed within 1 hours after a 10 minute exposure to the composition, more preferably at least 75% of ectoparasites present are killed within 30 minutes after a 10 minute exposure to the composition, even more preferably at least 75% of ectoparasites present are killed within 5 minutes after a 5 minute exposure to the composition.

By the term "killed" it is meant that ectoparasites, for instance lice, do not show any major vital signs. The criterion for the ectoparasiticidal efficacy (pediculicidal in the case of lice) of the test preparations was the mortality rate within each group of parasites - assessed by strictly defined criteria for vitality. Only when there was a total absence of vital signs or when there were only minimal vital signs such as gut movements or movements of the antennae ("minor vital signs") the parasites were declared to be dead. In the same manner, ovicidal efficacy was evaluated when there is no hatch at this stage.

In a preferred embodiment of the composition as herein disclosed, at least 80% of ectoparasites present are killed within 5 min after a 5 minute exposure to the composition, preferably at least 90% of ectoparasites present are killed within 5 minutes after a 5 minute exposure to the composition, more preferably at least 100% of ectoparasites present are killed within 5 minutes after a 5 minute exposure to the composition.

In a more preferred embodiment of the composition as herein disclosed, at least 80% of ectoparasites present are killed within 5 min after a 2 minute exposure to the composition, preferably at least 90% of ectoparasites present are killed within 5 minutes after a 2 minute exposure to the composition, more preferably at least 100% of ectoparasites present are killed within 5 minutes after a 2 minute exposure to the composition.

In a preferred embodiment, the ectoparasites are adult lice.

The compositions as herein disclosed are also highly effective in killing the ectoparasites ova. In a further embodiment of the composition as herein disclosed, at least 60% of the ova are killed within 14 days after 10 minute exposure, preferably at least 80% of the ova are killed within 14 days after 10 minute exposure, more preferably at least 80% of the ova are killed within 14 days after 5 minute exposure, even more preferably at least 40% of the ova are killed within 14 days after 2 minute exposure.

In a third aspect of the first invention, it is provided a topical composition comprising squalane and at least a siloxane.

In a further embodiment of the topical composition of the third aspect, squalane is present in an amount of at least 30% by weight in respect of the total amount of the composition, preferably in an amount of at least 40 % by weight in respect of the total amount of the composition, more preferably in an amount of at least 50 % by weight in respect of the total amount of the composition, even more preferably in an amount of at least 60 % by weight in respect of the total amount of the composition.

In a further embodiment of the topical composition as herein disclosed, the siloxane is a cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane. In a preferred embodiment, the total amount of siloxane or siloxanes present in the composition ranges from 30 to 70 % by weight in respect of the total amount of the composition, preferably ranges from 35 to 65 % by weight in respect of the total amount of the composition, more preferably ranges from 40 to 55 % by weight in respect of the total amount of the composition.

In a further embodiment of the topical composition as herein disclosed, the composition comprises at least a further emollient. Said further emollient is preferably a fatty acid ester, more preferably selected from cetyl palmitate, decyl oleate, ethyl oleate, isopropyl myristate, isopropyl palmitate, ethylhexyl stearate and mixtures thereof, even more preferably the composition comprises ethylhexyl stearate. In a preferred embodiment, the total amount of fatty acid ester or fatty acid esters present in the composition ranges from 1 to 10 % by weight in respect of the total amount of the composition, preferably ranges from 3 to 7 % by weight in respect of the total amount of the composition.

In a further embodiment of the topical composition as herein disclosed, said composition further comprises tea tree oil. In a preferred embodiment, the total amount of tea tree oil present in the composition ranges from 0.5 to 5% % by weight in respect of the total amount of the composition, preferably ranges from 1 to 3 % by weight in respect of the total amount of the composition.

The most preferred formulations of the present invention are comprised of a 50:45:5 mixture of a squalane, cyclomethicone and ethylhexyl stearate (w/w).

In a further embodiment of the topical composition as herein disclosed, the composition is for use in killing ectoparasites and/or their ova on a subject. In a preferred embodiment, squalane is the sole ectoparasiticidal agent present in the composition.

In a fourth aspect of the first invention, it is provided a method of killing ectoparasites and/or their ova on a subject comprising, topically administering squalane to an area on the subject where ectoparasites or their ova are present.

In a fifth aspect of the first invention, it is provided a method of killing ectoparasites and/or their ova on a subject comprising, topically administering to an area on the subject where ectoparasites or their ova are present the composition of the second aspect of the first invention.

The methods disclosed herein include topically administering a composition of the invention to an area on the human where ectoparasites are present. As noted above, the compositions preferably remain in contact with the treated area for a period of time.

The term "topical application" is meant that the composition is applied to the exterior of the treated subject, e.g. to the exterior skin, hair, fur, or foliage. This application includes, but is not limited to, manual application or application by various automated means, for example, spraying or painting onto a treated subject, or other means. In some embodiments, an ectoparasiticidal composition, for instance a pediculicidal composition, according to the present invention may be applied to the hair or skin of a subject in need thereof. The ectoparasiticidal composition, for instance a pediculicidal composition, may be applied topically in the form of a solution, cream, emulsion, lotion, gel, spray, ointment or foam.

In a further embodiment of the method of the fourth and fifth aspects, the composition is applied for 30 minutes or less, preferably the composition is applied for 20 minutes or less, more preferably the composition is applied for 10 minutes or less, even more preferably the composition is applied for 5 min or less. In a preferred embodiment, the composition is applied for 2 minutes.

In a further embodiment of the fourth and fifth aspects, the ectoparasites are non-flying ectoparasites. Said ectoparasites are preferably selected from the group consisting of lice, mites, ticks and fleas. In a preferred embodiment, the subject is a mammal. Said mammal is a human. In a more preferred embodiment, the ectoparasites are head lice.

In a sixth aspect of the first invention, it is provided the use of squalane for the manufacture of a medicament for killing ectoparasites and/or their ova on a subject.

In a seventh aspect of the first invention, it is provided the use of the composition of the second aspect of the first invention for the manufacture of a medicament for killing ectoparasites and/or their ova on a subject.

In an eighth aspect of the first invention, it is provided a kit for use in treating ectoparasite infestations comprising squalane or the composition of the second or third aspect of the first invention in a package or other enclosure; and instructions describing how to use the items included in the kit to kill ectoparasites.

The present invention also provides kits for treating ectoparasite infestations. In various embodiments, the kits include a composition of the present invention in a package or other enclosure. In a further embodiment of the kit of the eighth aspect, the kit further comprises a nit comb to assist in removing ectoparasites (e.g. lice) and their eggs from hair. The "nit comb" is an ordinary comb for ordering hair by passing it through the hair. For example, the OTC ANTIPIOJOS® nit comb (Ferrer Internacional SA, Spain), MEDI-SWEEP Lice Comb (Classic Products, Oxnard, CA) are preferred embodiments of the lice comb to be included in the kit. The package can be a box, or may simply be a wrapping (preferably of carton) that surrounds the kit. The comb is preferably provided inside the package, but can also be attached to the outside of the package. In other embodiments, the kits include shower cap. In preferred embodiments, the kit also contains instructions that describe how to use the items included in the kit to kill ectoparasites.

The term "nit" refers to an egg without embryo or a dead egg. With respect to eggs, this rather broad definition includes the following: i) viable eggs that will eventually hatch; ii) remnants of already-hatched eggs (nits) and, iii) nonviable eggs (dead embryo) that will never hatch.

A second invention provides hydrocarbon derivatives and fluid to semi-solid alkanes for killing ectoparasites and/or their ova on a subject. It also provides compositions and methods for killing ectoparasites on a subject. In the most preferred embodiments, the subject is a human and the ectoparasites are lice, fleas, mites and ticks.

The inventors discovered unexpectedly that aliphatic hydrocarbons are particularly effective in killing ectoparasites on a subject as well as their ova. The present inventors also discovered that the combination said aliphatic hydrocarbons and silicones (e.g., a cyclomethicone) also can kill ectoparasites. Said compounds may be included in a composition in an amount effective for killing lice, fleas, ticks, mites and other ectoparasites to result in a composition that is effective for this purpose.

In a first aspect of the second invention, it is provided an aliphatic hydrocarbon compound having a density of 0.7 to 0.9 $g/cm^3$ at 20 °C for use in killing non-flying ectoparasites and/or their ova on a subject.

In a further embodiment of the first aspect of the second invention, the hydrocarbon is preferably a fluid to semi-solid alkane. Said hydrocarbon is preferably selected from liquid paraffin, solid paraffin, petroleum jelly, squalane, squalene, isohexadecane, isoeicosane, isododecane and mixtures thereof. More preferably, the hydrocarbon is squalane.

In a further embodiment of the first aspect of the second invention, the ectoparasites are selected from the group consisting of lice, mites, ticks and fleas. In a preferred embodiment, the subject is a mammal, preferably the mammal is a human. In another preferred embodiment, the ectoparasites are head lice.

In a second aspect of the second invention, it is provided a composition for use in killing non-flying ectoparasites and/or their ova on a subject comprising an aliphatic hydrocarbon compound having a density of 0.7 to 0.9 $g/cm^3$ at 20 °C and at least a siloxane having a viscosity of less than 90 cSt.

The present invention provides compositions that are useful for treating ectoparasites on a mammal. The inventors discovered unexpectedly that just an aliphatic hydrocarbon compound having a density of 0.7 to 0.9 $g/cm^3$ at 20 °C and at least a siloxane having a viscosity less than 90 cSt. have the effect of killing ectoparasites and/or their ova.

The term "density" of a substance refers to the mass of a unit volume of a substance at 20°C. Density is usually listed in grams per cubic centimeter.

The viscosity of a fluid is a measure of its resistance to gradual deformation by shear stress or tensile stress. For liquids, it corresponds to the informal concept of "thickness". The term "viscosity" refers to the value measured at 25°C. The term viscosity in the present invention it refers to kinematic viscosity. The term "kinematic viscosity" refers to a viscosity which is determined by measuring the dynamic viscosity μ, and dividing the dynamic viscosity μ by the density of the fluid p, of the liquid measured at the same temperature. The unit of kinematic viscosity can be expressed by centistokes (cSt) equalling 1 mm2 /sec. Kinematic viscosity can be determined using the methods according to European. Pharmacopeia (Ph. Eur.) online 9.3, chapter (2.2.9) Capillary viscometer method and (2.2.49) Falling ball and automatic rolling ball viscometer methods.

In a further embodiment of the composition of second aspect of the second invention, the hydrocarbon compound is present in an amount of at least 5 % by weight in respect of the total amount of the composition. In a preferred embodiment, the hydrocarbon compound is present in an amount of at least 20% by weight in respect of the total amount of the composition, preferably in an amount of at least 30% by weight in respect of the total amount of the composition, preferably in an amount of at least 40 % by weight in respect of the total amount of the composition, more preferably in an amount of at least 50 % by weight in respect of the total amount of the composition and even more preferably, even more preferably in an amount of at least 60 % by weight in respect of the total amount of the composition.

In a further embodiment of the second aspect of the second invention, the ectoparasites are selected from the group consisting of lice, mites, ticks and fleas. In a preferred embodiment, the subject is a mammal, preferably the mammal is a human. In another preferred embodiment, the ectoparasites are head lice.

In a further embodiment of the second aspect of the second invention, the hydrocarbon is preferably a fluid to semi-solid alkane. Said hydrocarbon compound is preferably selected from liquid paraffin, solid paraffin, petroleum jelly, squalane, squalene, isohexadecane, isoeicosane, isododecane and mixtures thereof. Preferably, the hydrocarbon is squalane.

In a further embodiment of the composition of the second invention, the low-viscosity siloxane is a cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane. In a preferred embodiment, the total amount of siloxane or siloxanes present in the composition ranges from 30 to 70 % by weight in respect of the total amount of the composition, preferably ranges from 35 to 65 % by weight in respect of the total amount of the composition, more preferably ranges from 40 to 55 % by weight in respect of the total amount of the composition.

In a further embodiment of the composition of the second invention, the composition comprises at least a further emollient. Said further emollient is preferably a fatty acid ester, more preferably selected from cetyl palmitate, decyl oleate, ethyl oleate, isopropyl myristate, isopropyl palmitate, ethylhexyl stearate and mixtures thereof, even more preferably the composition comprises ethylhexyl stearate.

In a preferred embodiment of the composition of the second invention, the total amount of fatty acid ester or fatty acid esters present in the composition ranges from 1 to 10 % by weight in respect of the total amount of the composition, preferably ranges from 3 to 7 % by weight in respect of the total amount of the composition.

In a further embodiment of the composition of the second invention, said composition further comprises tea tree oil. In a preferred embodiment, the total amount of tea tree oil present in the composition ranges from 0.5 to 5% % by weight in respect of the total amount of the composition, preferably ranges from 1 to 3 % by weight in respect of the total amount of the composition.

In a further embodiment of the composition of the second invention, at least 75% of ectoparasites present are killed within 4 hours after a 10 minute exposure to the composition, preferably at least 75% of ectoparasites present are killed within 2 hours after a 10 minute exposure to the composition, preferably at least 75% of ectoparasites present are killed within 1 hours after a 10 minute exposure to the composition, more preferably at least 75% of ectoparasites present are killed within 30 minutes after a 10 minute exposure to the composition, even more preferably at least 75% of ectoparasites present are killed within 5 minutes after a 5 minute exposure to the composition.

In a preferred embodiment of the composition of the second invention, at least 80% of ectoparasites present are killed within 5 min after a 5 minute exposure to the composition, preferably at least 90% of ectoparasites present are killed within 5 minutes after a 5 minute exposure to the composition, more preferably at least 100% of ectoparasites present are killed within 5 minutes after a 5 minute exposure to the composition.

In a more preferred embodiment of the composition of the second invention, at least 80% of ectoparasites present are killed within 5 min after a 2 minute exposure to the composition, preferably at least 90% of ectoparasites present are killed within 5 minutes after a 2 minute exposure to the composition, more preferably at least 100% of ectoparasites present are killed within 5 minutes after a 2 minute exposure to the composition.

In a preferred embodiment of the composition of the second invention, the ectoparasites are adult lice.

The compositions of the second invention are also highly effective in killing the ectoparasites ova. In a further embodiment of the composition as herein disclosed, at least 60% of the ova are killed within 14 days after 10 minute exposure, preferably at least 80% of the ova are killed within 14 days after 10 minute exposure, more preferably at least 80% of the ova are killed within 14 days after 5 minute exposure, even more preferably at least 40% of the ova are killed within 14 days after 2 minute exposure.

In a third aspect of the second invention, it is provided a method of killing non-flying ectoparasites and/or their ova on a subject comprising, topically administering to an area on the subject where ectoparasites or their ova are present a composition of the second aspect of the second invention.

In a further embodiment of the method of the third aspect of the second invention, the composition is applied for 30 minutes or less, preferably the composition is applied for 20 minutes or less, more preferably the composition is applied for 10 minutes or less, even more preferably the composition is applied for 5 min or less. In a preferred embodiment, the composition is applied for 2 minutes.

In a further embodiment of the method of the third aspect of the second invention, the ectoparasites are non-flying ectoparasites. Said ectoparasites are preferably selected from the group consisting of lice, mites, ticks and fleas. In a preferred embodiment, the subject is a mammal. Said mammal is a human. In a more preferred embodiment, the ectoparasites are head lice.

In a fourth aspect of the second invention, it is provided the use of an aliphatic hydrocarbon compound having a density of 0.7 to 0.9 g/cm$^3$ at 20 °C for the manufacture of a medicament for killing ectoparasites and/or their ova on a subject.

In a fifth aspect of the second invention, it is provided the use of the composition of the second aspect of the second invention for the manufacture of a medicament for killing ectoparasites and/or their ova on a subject.

In a sixth aspect of the second invention, it is provided a kit for use in treating ectoparasite infestations comprising, the composition of the second aspect of the second invention in a package or other enclosure; and instructions describing how to use the items included in the kit to kill ectoparasites.

The second invention also provides kits for treating ectoparasite infestations. In various embodiments, the kits include a composition of the present invention in a package or other enclosure. In a further embodiment of the kit of the sixth aspect of the second invention, the kit further comprises a nit comb to assist in removing ectoparasites (e.g. lice) and their eggs from hair. The package can be a box, or may simply be a wrapping (preferably of a plastic) that surrounds the kit. The comb is preferably provided inside the package, but can also be attached to the outside of the package. In other embodiments, the kit includes shower cap. In preferred embodiments, the kit also contains instructions that describe how to use the items included in the kit to kill ectoparasites.

A third invention provides a composition for killing ectoparasites and/or their ova on a subject. It also provides compositions and methods for killing ectoparasites on a subject. In the most preferred embodiments, the subject is a human and the ectoparasites are lice, fleas, and ticks.

The inventors discovered unexpectedly that a fatty acid ester in combination with at least an essential oil is a composition particularly effective in killing ectoparasites on a subject as well as their ova. Said compounds may be included in a composition in an amount effective for killing lice, fleas, ticks and other ectoparasites to result in a composition that is effective for this purpose.

In a first aspect of the third invention, it is provided a composition for use in killing non-flying ectoparasites and/or their ova on a subject comprising at least a fatty acid ester and at least an essential oil.

In a further embodiment of the first aspect of the third invention, the composition does not comprise Lippia javanica essential oil.

In a further embodiment of the first aspect of the third invention, the ectoparasites are selected from the group consisting of lice, mites, ticks and fleas. In a preferred embodiment, the subject is a mammal, preferably the mammal is a human. In another preferred embodiment, the ectoparasites are head lice.

In a further embodiment of the first aspect of the third invention, the fatty acid ester is an ester of a fatty acid selected from myristate, laurate, palmitate, stearate, arachidate, behenate, lignocerate, palmitoleate, oleate, linoleate, linolenate and arachidonate and mixtures thereof, preferably the composition comprises isopropyl myristate. In a preferred embodiment, the total amount of fatty acid ester or fatty acid esters present in the composition ranges from 30 to 65 % by weight in respect of the total amount of the composition, preferably ranges from 35 to 55 % by weight in respect of the total amount of the composition, more preferably ranges from 45 to 55 % by weight in respect of the total amount of the composition.

In a further embodiment of the first aspect of the third invention, the essential oil is selected from eucalyptus oil, lemongrass oil, petitgrain oil, Rosmarinum officinalis (rosemary) oil, Thymus vulgaris (thyme) oil, Lavendula augustifolia (lavender) oil, Melaleuca alternifolia (tea tree) oil, Tagete minuta (marigold) oil, Levisticum officinalis (lovage) oil, cinnamon oil, lemon oil, orange oil, grapefruit oil, oil of bergamot and mixtures thereof, preferably the composition comprises tea tree oil. In a preferred embodiment, the total amount of essential oil or essential oils present in the composition ranges from 0.2 to 5% % by weight in respect of the total amount of the composition, preferably ranges from 0.5 to 3 % by weight in respect of the total amount of the composition.

In a further embodiment of the first aspect of the third invention, the composition further comprises at least a siloxane. Said siloxane is preferably a cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane. In a preferred embodiment, wherein the total amount of siloxane or siloxanes present in the composition ranges from 30 to 60 % by weight in respect of the total amount of the composition, preferably ranges from 35 to 55 % by weight in respect of the total amount of the composition, more preferably ranges from 40 to 50 % by weight in respect of the total amount of the composition.

In a further embodiment of the first aspect of the third invention, the composition further comprises ethylhexyl stearate. In a preferred embodiment, the total amount of ethylhexyl stearate present in the composition ranges from 1 to 8 % by weight in respect of the total amount of the composition, preferably ranges from 2 to 6 % by weight in respect of the total amount of the composition.

In a further embodiment of the first aspect of the third invention, the composition comprises, 45 to 55 % w/w of isopropyl myristate, 40 to 50 % w/w decamethylcyclopentasiloxane, 2 to 6 % w/w ethylhexyl stearate and 0.5 to 3 % w/w Melaleuca alternifolia (tea tree) oil.

In a further embodiment of the composition of the third invention, at least 75% of ectoparasites present are killed within 4 hours after a 10 minute exposure to the composition, preferably at least 75% of ectoparasites present are killed within 2 hours after a 10 minute exposure to the composition, preferably at least 75% of ectoparasites present are killed within 1 hours after a 10 minute exposure to the composition, more preferably at least 75% of ectoparasites present are killed within 30 minutes after a 10 minute exposure to the composition, even more preferably at least 75% of ectoparasites present are killed within 5 minutes after a 5 minute exposure to the composition.

In a preferred embodiment of the composition of the third invention, at least 80% of ectoparasites present are killed within 5 min after a 5 minute exposure to the composition, preferably at least 90% of ectoparasites present are killed within 5 minutes after a 5 minute exposure to the composition, more preferably at least 100% of ectoparasites present are killed within 5 minutes after a 5 minute exposure to the composition.

In a preferred embodiment of the composition of the third invention, the ectoparasites are adult lice.

The compositions of the third invention are also highly effective in killing the ectoparasites ova. In a further embodiment of the composition as herein disclosed, at least 80% of the ova are killed within 14 days after 15 minute exposure, preferably at least 50% of the ova are killed within 14 days after 5 minute exposure.

In a second aspect of the third invention, it is provided a method of killing non-flying ectoparasites and/or their ova on a subject comprising, topically administering to an area on the subject where ectoparasites or their ova are present the composition of the first aspect of the third invention.

In a further embodiment of the method of the second aspect of the third invention, the composition is applied for 30 minutes or less, preferably the composition is applied for 20 minutes or less, more preferably the composition is applied for 10 minutes or less, even more preferably the composition is applied for 5 min or less.

In a further embodiment of the method of the second aspect of the third invention, the ectoparasites are non-flying ectoparasites. Said ectoparasites are preferably selected from the group consisting of lice, mites, ticks and fleas. In a preferred embodiment, the subject is a mammal. Said mammal is a human. In a more preferred embodiment, the ectoparasites are head lice.

In a third aspect of the third invention, it is provided the use of the composition of the first aspect of the third invention for the manufacture of a medicament for killing ectoparasites and/or their ova on a subject.

In a fourth aspect of the third invention, it is provided a kit for use in treating ectoparasite infestations comprising the composition of the first aspect of the third invention in a package or other enclosure; and instructions describing how to use the items included in the kit to kill ectoparasites

The third invention also provides kits for treating ectoparasite infestations. In various embodiments, the kits include a composition of the present invention in a package or other enclosure. In a further embodiment of the kit of the fourth aspect of the third invention, the kit further comprises a nit comb to assist in removing ectoparasites (e.g. lice) and their eggs from hair. The package can be a box, or may simply be a wrapping (preferably of a plastic) that surrounds the kit. The comb is preferably provided inside the package, but can also be attached to the outside of the package. In other embodiments, the kit includes shower cap. In preferred embodiments, the kit also contains instructions that describe how to use the items included in the kit to kill ectoparasites.

Further aspects and embodiments of the present invention are described in the following clauses below:

Clause 1.- Squalane for use as a medicament.

Clause 2.- The product according to clause 1 for use in killing ectoparasites and/or their ova on a subject.

Clause 3.- The product for use according to clause 2, wherein the ectoparasites are non-flying ectoparasites.

Clause 4.- The product for use according to clause 3, wherein the ectoparasites are selected from the group consisting of lice, mites, ticks and fleas.

Clause 5.- The product for use according to any one of clauses 2 to 4, wherein the subject is a mammal or plant, preferably the subject is a mammal.

Clause 6.- The product for use according to clause 5, wherein the mammal is a human.

Clause 7.- The product for use according to any one of clauses 4 to 6, wherein the ectoparasites are head lice.

Clause 8.- A composition for use in killing ectoparasites and/or their ova on a subject comprising squalane, wherein squalane is present in an amount of at least 5 % by weight in respect of the total amount of the composition.

Clause 9.- The composition for use according to the preceding clause, wherein the composition does not comprise Lippia javanica essential oil.

Clause 10.- The composition for use according to any one of clauses 8 to 9, wherein the total amount of squalane present in the composition ranges from 5 to 20 % by weight in respect of the total amount of the composition.

Clause 11.- The composition for use according to clause 8, wherein squalane is present in an amount of more than 20 % by weight in respect of the total amount of the composition.

Clause 12.- The composition for use according to any one of clauses 8 to 11, wherein the ectoparasites are selected from the group consisting of lice, mites, ticks and fleas.

Clause 13.- The composition for use according to any one of clauses 8 to 12, wherein the subject is a mammal.

Clause 14.- The composition for use according to clause 13, wherein the mammal is a human.

Clause 15.- The composition for use according to any one of clauses 12 to 14, wherein the ectoparasites are head lice.

Clause 16.- The composition for use according to any one of clauses 11 to 15, wherein squalane is present in an amount of at least 30% by weight in respect of the total amount of the composition, preferably in an amount of at least 40 % by weight in respect of the total amount of the composition, more preferably in an amount of at least 50 % by weight in respect of the total amount of the composition, even more preferably in an amount of at least 60 % by weight in respect of the total amount of the composition.

Clause 17.- The composition for use according to any one of clauses 8 to 16, wherein the composition further comprises at least a siloxane.

Clause 18.- The composition for use according to the preceding clause, wherein the siloxane is a cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane.

Clause 19.- The composition for use according to any one of the two preceding clauses, wherein the total amount of siloxane or siloxanes present in the composition ranges from 30 to 70 % by weight in respect of the total amount of the composition, preferably ranges from 35 to 65 % by weight in respect of the total amount of the composition, more preferably ranges from 40 to 55 % by weight in respect of the total amount of the composition.

Clause 20.- The composition for use according to any one of clauses 8 to 19, wherein the composition comprises at least a further emollient.

Clause 21.- The composition for use according to the preceding clause, wherein the further emollient is a fatty acid ester preferably selected from cetyl palmitate, decyl oleate, ethyl oleate, isopropyl myristate, isopropyl palmitate, ethylhexyl stearate (i.e., 2-ethylhexyl stearate) and mixtures thereof, more preferably the composition comprises ethylhexyl stearate.

Clause 22.- The composition for use according to the preceding clause, wherein the total amount of fatty acid ester or fatty acid esters present in the composition ranges from 1 to 10 % by weight in respect of the total amount of the composition, preferably ranges from 3 to 7 % by weight in respect of the total amount of the composition.

Clause 23.- The composition for use according to any one of clauses 8 to 22, wherein the composition further comprises tea tree oil.

Clause 24.- The composition for use according to the preceding clause, wherein the total amount of tea tree oil present in the composition ranges from 0.5 to 5% % by weight in respect of the total amount of the composition, preferably ranges from 1 to 3 % by weight in respect of the total amount of the composition.

Clause 25.- The composition for use according to clause 16, wherein the composition consists only of squalane.

Clause 26.- The composition for use according to any one of clauses 8 to 25, wherein at least 75% of ectoparasites present are killed within 4 hours after a 10 minute exposure to the composition.

Clause 27.- The composition for use according to any one of clauses 8 to 26, wherein at least 75% of ectoparasites present are killed within 2 hours after a 10 minute exposure to the composition.

Clause 28.- The composition for use according to any one of clauses 8 to 27, wherein at least 75% of ectoparasites present are killed within 1 hours after a 10 minute exposure to the composition.

Clause 29.- The composition for use according to any one of clauses 8 to 28, wherein at least 75% of ectoparasites present are killed within 30 minutes after a 10 minute exposure to the composition.

Clause 30.- The composition for use according to any one of clauses 8 to 29, wherein at least 75% of ectoparasites present are killed within 10 minutes after a 5 minute exposure to the composition.

Clause 31.- The composition for use according to any one of clauses 8 to 30, wherein at least 75% of ectoparasites present are killed within 5 minutes after a 5 minute exposure to the composition.

Clause 32.- The composition for use according to any one of clauses 8 to 31, wherein at least 80% of ectoparasites present are killed within 5 min after a 5 minute exposure to the composition.

Clause 33.- The composition for use according to any one of clauses 8 to 32, wherein at least 90% of ectoparasites present are killed within 5 minutes after a 5 minute exposure to the composition.

Clause 34.- The composition for use according to any one of clauses 8 to 33, wherein at least 100% of ectoparasites present are killed within 5 minutes after a 5 minute exposure to the composition.

Clause 35.- The composition for use according to any one of clauses 8 to 34, wherein the ectoparasites are adult lice.

Clause 36.- The composition for use according to any one of clauses 8 to 35, wherein at least 60% of the ova are killed within 14 days after 10 minute exposure.

Clause 37.- The composition for use according to any one of clauses 8 to 36, wherein at least 80% of the ova are killed within 14 days after 10 minute exposure.

Clause 38.- The composition for use according to any one of clauses 8 to 37, wherein at least 80% of the ova are killed within 14 days after 5 minute exposure.

Clause 39.- A topical composition comprising squalane and at least a siloxane.

Clause 40.- The composition according to clause 39, wherein squalane is present in an amount of at least 30% by weight in respect of the total amount of the composition, preferably in an amount of at least 40 % by weight in respect of the total amount of the composition, more preferably in an amount of at least 50 % by weight in respect of the total amount of the composition, even more preferably in an amount of at least 60 % by weight in respect of the total amount of the composition.

Clause 41.- The composition according to any one of clauses 39 to 40, wherein the siloxane is a cyclic siloxane, preferably the cyclic siloxane is selected from cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane.

Clause 42.- The composition according to any one of clauses 39 to 41, wherein the total amount of siloxane or siloxanes present in the composition ranges from 30 to 70 % by weight in respect of the total amount of the composition, preferably ranges from 35 to 65 % by weight in respect of the total amount of the composition, more preferably ranges from 40 to 55 % by weight in respect of the total amount of the composition.

Clause 43.- The composition according to any one of clauses 39 to 42, wherein the composition comprises at least a further emollient.

Clause 44.- The composition according to clause 43, wherein the further emollient is a fatty acid ester preferably selected from cetyl palmitate, decyl oleate, ethyl oleate, isopropyl myristate, isopropyl palmitate, ethylhexyl stearate and mixtures thereof, more preferably the composition comprises ethylhexyl stearate.

Clause 45.- The composition according to clause 44, wherein the total amount of fatty acid ester or fatty acid esters present in the composition ranges from 1 to 10 % by weight in respect of the total amount of the composition, preferably ranges from 3 to 7 % by weight in respect of the total amount of the composition.

Clause 46.- The composition according to anyone of clauses 39 to 45, wherein the composition further comprises tea tree oil.

Clause 47.- The composition according to the preceding clause, wherein the total amount of tea tree oil present in the composition ranges from 0.5 to 5% % by weight in respect of the total amount of the composition, preferably ranges from 1 to 3 % by weight in respect of the total amount of the composition.

Clause 48.- The composition according to any one of clauses 39 to 47, wherein the composition is for use in killing ectoparasites and/or their ova on a subject.

Clause 49.- The composition according to any one of clauses 8 to 48, wherein squalane is the sole ectoparasiticidal agent present in the composition.

Clause 50.- A method of killing ectoparasites and/or their ova on a subject comprising, topically administering squalane to an area on the subject where ectoparasites or their ova are present.

Clause 51.- A method of killing ectoparasites and/or their ova on a subject comprising, topically administering to an area on the subject where ectoparasites or their ova are present a composition as defined in any one of the clauses 8 to 49.

Clause 52.- The method according to any one of clauses 50 to 51, wherein the composition is applied for 30 minutes or less.

Clause 53.- The method according to any one of clauses 50 to 52, wherein the composition is applied for 20 minutes or less.

Clause 54.- The method according to any one of clauses 50 to 53, wherein the composition is applied for 10 minutes or less.

Clause 55.- The method according to any one of clauses 50 to 54, wherein the composition is applied for 5 min or less.

Clause 56.- The method according to any one of clauses 50 to 55, wherein the ectoparasites are non-flying ectoparasites.

Clause 57.- The method according to any one of clauses 50 to 56, wherein the ectoparasites are selected from the group consisting of lice, mites, ticks and fleas.

Clause 58.- The method according to any one of clauses 50 to 57, wherein the subject is a mammal.

Clause 59.- The method according to clause 58, wherein the mammal is a human.

Clause 60.- The method according to any one of clauses 50 to 59, wherein the ectoparasites are head lice.

Clause 61.- Use of squalane for the manufacture of a medicament for killing ectoparasites and/or their ova on a subject.

Clause 62.- Use of a composition as defined in any one of the clauses 8 to 49 for the manufacture of a medicament for killing ectoparasites and/or their ova on a subject.

Clause 63.- A kit for use in treating ectoparasite infestations comprising squalane or a composition as defined in any one of clauses 8 to 49 in a package or other enclosure; and instructions describing how to use the items included in the kit to kill ectoparasites.

Clause 64.- The kit for use according to clause 63 further comprising a nit comb.

Clause 65.- An aliphatic hydrocarbon compound having a density of 0.7 to 0.9 $g/cm^3$ at 20 °C for use in killing non-flying ectoparasites and/or their ova on a subject.

Clause 66.- The hydrocarbon compound for use according to the preceding clause, wherein said hydrocarbon is preferably a fluid to semi-solid alkane.

Clause 67.- The hydrocarbon compound for use according to any one of clauses 65 to 66, wherein the hydrocarbon is selected from liquid paraffin, solid paraffin, petroleum jelly, squalane, squalene, isohexadecane, isoeicosane, isododecane and mixtures thereof.

Clause 68.- The hydrocarbon compound for use according to any one of clauses 65 to 67, wherein the hydrocarbon is squalane.

Clause 69.- The hydrocarbon compound for use according to any one of clauses 65 to 68, wherein the ectoparasites are selected from the group consisting of lice, mites, ticks and fleas.

Clause 70.- The hydrocarbon compound for use according to any one of clauses 65 to 69, wherein the subject is a mammal.

Clause 71.- The hydrocarbon compound for use according to any one of clauses 65 to 70, wherein the mammal is a human.

Clause 72.- The hydrocarbon compound for use according to any one of clauses 65 to 71, wherein the ectoparasites are head lice.

Clause 73.- A composition for use in killing non-flying ectoparasites and/or their ova on a subject comprising an aliphatic hydrocarbon compound having a density of 0.7 to 0.9 g/cm$^3$ at 20 °C and at least a siloxane having a viscosity less than 90 cSt.

Clause 74.- The composition for use according to the preceding clause, wherein the hydrocarbon compound is present in an amount of at least 5 % by weight in respect of the total amount of the composition.

Clause 75.- The composition for use according to any one of clauses 73 to 74, wherein the ectoparasites are selected from the group consisting of lice, mites, ticks and fleas.

Clause 76.- The composition for use according to any one of clauses 73 to 75, wherein the subject is a mammal.

Clause 77.- The composition for use according to any one of clauses 73 to 76, wherein the mammal is a human.

Clause 78.- The composition for use according to any one of clauses 73 to 77, wherein the ectoparasites are head lice.

Clause 79.- The composition for use according to any one of clauses 73 to 78, wherein the hydrocarbon compound is present in an amount of at least 20% by weight in respect of the total amount of the composition, preferably in an amount of at least 30% by weight in respect of the total amount of the composition, preferably in an amount of at least 40 % by weight in respect of the total amount of the composition, more preferably in an amount of at least 50 % by weight in respect of the total amount of the composition and even more preferably, even more preferably in an amount of at least 60 % by weight in respect of the total amount of the composition.

Clause 80.- The composition for use according to any one of clauses 73 to 79, wherein the hydrocarbon is preferably a fluid to semi-solid alkane.

Clause 81.- The composition for use according to any one of clauses 73 to 80, wherein the hydrocarbon compound is selected from liquid paraffin, solid paraffin, petroleum jelly, squalane, squalene, isohexadecane, isoeicosane, isododecane and mixtures thereof.

Clause 82.- The composition for use according to any one of clauses 73 to 81, wherein the hydrocarbon is squalane.

Clause 83.- The composition for use according to any one of clauses 73 to 82, wherein the low-viscosity siloxane is a cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane.

Clause 84.- The composition for use according to any one of clauses 73 to 83, wherein the total amount of siloxane or siloxanes present in the composition ranges from 30 to 70 % by weight in respect of the total amount of the composition, preferably ranges from 35 to 65 % by weight in respect of the total amount of the composition, more preferably ranges from 40 to 55 % by weight in respect of the total amount of the composition.

Clause 85.- The composition for use according to any one of clauses 73 to 84, wherein the composition comprises at least a further emollient.

Clause 86.- The composition for use according to the preceding clause, wherein the further emollient is a fatty acid ester preferably selected from cetyl palmitate, decyl oleate, ethyl oleate, isopropyl myristate, isopropyl palmitate, ethylhexyl stearate and mixtures thereof, more preferably the composition comprises ethylhexyl stearate.

Clause 87.- The composition for use according to the preceding clause, wherein the total amount of fatty acid ester or fatty acid esters present in the composition ranges from 1 to 10 % by weight in respect of the total amount of the composition, preferably ranges from 3 to 7 % by weight in respect of the total amount of the composition.

Clause 88.- The composition for use according to any one of clauses 73 to 87, wherein the composition further comprises tea tree oil.

Clause 89.- The composition for use according to the preceding clause, wherein the total amount of tea tree oil present in the composition ranges from 0.5 to 5% % by weight in respect of the total amount of the composition, preferably ranges from 1 to 3 % by weight in respect of the total amount of the composition.

Clause 90.- The composition for use according to any one of clauses 73 to 89, wherein at least 75% of ectoparasites present are killed within 4 hours after a 10 minute exposure to the composition.

Clause 91.- The composition for use according to any one of clauses 73 to 90, wherein at least 75% of ectoparasites present are killed within 2 hours after a 10 minute exposure to the composition.

Clause 92.- The composition for use according to any one of clauses 73 to 91, wherein at least 75% of ectoparasites present are killed within 1 hours after a 10 minute exposure to the composition.

Clause 93.- The composition for use according to any one of clauses 73 to 92, wherein at least 75% of ectoparasites present are killed within 30 minutes after a 10 minute exposure to the composition.

Clause 94.- The composition for use according to any one of clauses 73 to 93, wherein at least 75% of ectoparasites present are killed within 10 minutes after a 5 minute exposure to the composition.

Clause 95.- The composition for use according to any one of clauses 73 to 94, wherein at least 75% of ectoparasites present are killed within 5 minutes after a 5 minute exposure to the composition.

Clause 96.- The composition for use according to any one of clauses 73 to 95, clauses wherein at least 80% of ectoparasites present are killed within 5 minutes after a 5 minute exposure to the composition.

Clause 97.- The composition for use according to any one of clauses 73 to 96, wherein at least 90% of ectoparasites present are killed within 5 minutes after a 5 minute exposure to the composition.

Clause 98.- The composition for use according to any one of clauses 73 to 97, wherein at least 100% of ectoparasites present are killed within 5 minutes after a 5 minute exposure to the composition.

Clause 99.- The composition for use according to any one of clauses 73 to 98, wherein the ectoparasites are adult lice.

Clause 100.- The composition for use according to any one of clauses 73 to 99, wherein at least 60% of the ova are killed within 14 days after 10 minute exposure.

Clause 101.- The composition for use according to any one of clauses 73 to 100, wherein at least 80% of the ova are killed within 14 days after 10 minute exposure.

Clause 102.- The composition for use according to any one of clauses 73 to 101, wherein at least 80% of the ova are killed within 14 days after 5 minute exposure.

Clause 103.- A method of killing non-flying ectoparasites and/or their ova on a subject comprising, topically administering to an area on the subject where ectoparasites or their ova are present a composition as defined in any one of the clauses 73 to 102.

Clause 104.- The method according to clause 103, wherein the composition is applied for 30 minutes or less.

Clause 105.- The method according to any one of clauses 103 to 104, wherein the composition is applied for 20 minutes or less.

Clause 106.- The method according to any one of clauses 103 to 105, wherein the composition is applied for 10 minutes or less.

Clause 107.- The method according to any one of clauses 103 to 106, wherein the composition is applied for 5 minutes or less.

Clause 108.- The method according to any one of clauses 103 to 107, wherein the ectoparasites are selected from the group consisting of lice, mites, ticks and fleas.

Clause 109.- The method according to any one of clauses 103 to 108, wherein the subject is a mammal.

Clause 110.- The method according to any one of clauses 103 to 109, wherein the mammal is a human.

Clause 111.- The method according to any one of clauses 103 to 110, wherein the ectoparasites are head lice.

Clause 112.- Use of an aliphatic hydrocarbon compound having a density of 0.7 to 0.9 g/cm$^3$ at 20 °C for the manufacture of a medicament for killing ectoparasites and/or their ova on a subject.

Clause 113.- Use of a composition as defined in any one of the clauses 73 to 102 for the manufacture of a medicament for killing ectoparasites and/or their ova on a subject.

Clause 114.- A kit for use in treating ectoparasite infestations comprising, a composition as defined in any one of clauses 73 to 102 in a package or other enclosure; and instructions describing how to use the items included in the kit to kill ectoparasites.

Clause 115.- The kit for use according to clause 114 further comprising a nit comb.

Clause 116.- A composition for use in killing non-flying ectoparasites and/or their ova on a subject comprising at least a fatty acid ester and at least an essential oil.

Clause 117.- The composition for use according to the preceding clause, wherein the composition does not comprise Lippia javanica essential oil.

Clause 118.- The composition for use according to any one of clauses 116 to 117, wherein the ectoparasites are selected from the group consisting of lice, mites, ticks and fleas.

Clause 119.- The composition for use according to any one of clauses 116 to 118, wherein the subject is a mammal.

Clause 120.- The composition for use according to any one of clauses 116 to 119, wherein the mammal is a human.

Clause 121.- The composition for use according to any one of clauses 116 to 120, wherein the ectoparasites are head lice.

Clause 122.- The composition for use according to any one of clauses 116 to 121, wherein the fatty acid ester is preferably an ester of a fatty acid selected from myristate, laurate, palmitate, stearate, arachidate, behenate, lignocerate, palmitoleate, oleate, linoleate, linolenate and arachidonate and mixtures thereof, more preferably the composition comprises isopropyl myristate.

Clause 123.- The composition for use according to any one of clauses 116 to 122, wherein the total amount of fatty acid ester or fatty acid esters present in the composition ranges from 30 to 65 % by weight in respect of the total amount of the composition, preferably ranges from 35 to 55 % by weight in respect of the total amount of the composition, more preferably ranges from 45 to 55 % by weight in respect of the total amount of the composition.

Clause 124.- The composition for use according to any one of clauses 116 to 123, wherein the essential oil is selected from eucalyptus oil, lemongrass oil, petitgrain oil, Rosmarinum officinalis (rosemary) oil, Thymus vulgaris (thyme) oil, Lavendula augustifolia (lavender) oil, Melaleuca alternifolia (tea tree) oil, Tagete minuta (marigold) oil, Levisticum officinalis (lovage) oil, cinnamon oil, lemon oil, orange oil, grapefruit oil, oil of bergamot and mixtures thereof, preferably the composition comprises tea tree oil.

Clause 125.- The composition for use according to any one of clauses 116 to 124, wherein the total amount of essential oil or essential oils present in the composition ranges from 0.2 to 5% % by weight in respect of the total amount of the composition, preferably ranges from 0.5 to 3 % by weight in respect of the total amount of the composition.

Clause 126.- The composition for use according to any one of clauses 116 to 125, wherein the composition further comprises at least a siloxane.

Clause 127.- The composition for use according to the preceding clause, wherein the siloxane is a cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane.

Clause 128.- The composition according to any one of clauses 126 to 127, wherein the total amount of siloxane or siloxanes present in the composition ranges from 30 to 60 % by weight in respect of the total amount of the composition, preferably ranges from 35 to 55 % by weight in respect of the total amount of the composition, more preferably ranges from 40 to 50 % by weight in respect of the total amount of the composition.

Clause 129.- The composition for use according to any one of clauses 116 to 128, wherein the composition further comprises ethylhexyl stearate.

Clause 130.- The composition for use according to the preceding clause, wherein the total amount of ethylhexyl stearate present in the composition ranges from 1 to 8 % by weight in respect of the total amount of the composition, preferably ranges from 2 to 6 % by weight in respect of the total amount of the composition.

Clause 131.- The composition for use according to any one of clauses 116 to 130, wherein the composition comprises,

45 to 55 % w/w of isopropyl myristate,
40 to 50 % w/w decamethylcyclopentasiloxane,
2 to 6 % w/w ethylhexyl stearate and,
0.5 to 3 % w/w Melaleuca alternifolia (tea tree) oil

Clause 132.- The composition for use according to any one of clauses 116 to 131, wherein at least 75% of ectoparasites present are killed within 4 hours after a 10 minute exposure to the composition.

Clause 133.- The composition for use according to any one of clauses 116 to 132, wherein at least 75% of ectoparasites present are killed within 2 hours after a 10 minute exposure to the composition.

Clause 134.- The composition for use according to any one of clauses 116 to 133, wherein at least 75% of ectoparasites present are killed within 1 hours after a 10 minute exposure to the composition.

Clause 135.- The composition for use according to any one of clauses 116 to 134, wherein at least 75% of ectoparasites present are killed within 30 minutes after a 10 minute exposure to the composition.

Clause 136.- The composition for use according to any one of clauses 116 to 135, wherein at least 75% of ectoparasites present are killed within 10 minutes after a 5 minute exposure to the composition.

Clause 137.- The composition for use according to any one of clauses 116 to 136, wherein at least 75% of ectoparasites present are killed within 5 minutes after a 5 minute exposure to the composition.

Clause 138.- The composition for use according to any one of clauses 116 to 137, wherein at least 80% of ectoparasites present are killed within 5 min after a 5 minute exposure to the composition.

Clause 139.- The composition for use according to any one of clauses 116 to 138, wherein at least 90% of ectoparasites present are killed within 5 minutes after a 5 minute exposure to the composition.

Clause 140.- The composition for use according to any one of clauses 116 to 139, wherein at least 100% of ectoparasites present are killed within 5 minutes after a 5 minute exposure to the composition.

Clause 141.- The composition for use according to any one of clauses 116 to 140 wherein the ectoparasites are adult lice.

Clause 142.- The composition for use according to any one of clauses 116 to 141, wherein at least 80% of the ova are killed within 14 days after 15 minute exposure.

Clause 143.- The composition for use according to any one of clauses 116 to 142, wherein at least 50% of the ova are killed within 14 days after 5 minute exposure.

Clause 144.- A method of killing non-flying ectoparasites and/or their ova on a subject comprising, topically administering to an area on the subject where ectoparasites or their ova are present a composition as defined in any one of the clauses 116 to 143.

Clause 145.- The method according to clause 144, wherein the composition is applied for 30 minutes or less.

Clause 146.- The method according to any one of clauses 144 to 145, wherein the composition is applied for 20 minutes or less.

Clause 147.- The method according to any one of clauses 144 to 146, wherein the composition is applied for 10 minutes or less.

Clause 148.- The method according to any one of clauses 144 to 147, wherein the composition is applied for 5 minutes or less.

Clause 149.- The method according to any one of clauses 144 to 148, wherein the ectoparasites are selected from the group consisting of lice, mites, ticks and fleas.

Clause 150.- The method according to any one of clauses 144 to 149, wherein the subject is a mammal.

Clause 151.- The method according to any one of clauses 144 to 150, wherein the mammal is a human.

Clause 152.- The method according to any one of clauses 144 to 151, wherein the ectoparasites are head lice.

Clause 153.- Use of a composition as defined in any one of the clauses 116 to 143 for the manufacture of a medicament for killing ectoparasites and/or their ova on a subject.

Clause 154.- A kit for use in treating ectoparasite infestations comprising, a composition as defined in any one of clauses 116 to 143 in a package or other enclosure; and instructions describing how to use the items included in the kit to kill ectoparasites.

Clause 155.- The kit for use according to clause 154 further comprising a nit comb.

**EXAMPLES**

The following examples illustrate various embodiment of the invention and are not intended to limit the invention in any way.

**Example 1 - Formulations with squalane**

Squalane, cyclomethicone and ethylhexyl stearate were blended in a recipient at room temperature (22°C ± 2°C) with a continuous mixing during about 20 minutes at low to medium speed to prevent incorporation of air. The product can be stored in an enclosed cover stainless steel storage tank at controlled room temperatures of 15-30°C until packaging.

| Ingredients | Compositions (w/w %) | | | | | |
|---|---|---|---|---|---|---|
| | Ex 1.1 | Ex 1.2 | Ex 1.3 | Ex 1.4 | Ex 1.5 | Ex 1.6 |
| Squalane | 50 | 100 | 30 | 40 | 20 | 75 |
| Decamethylcyclopentasiloxane | 45 | | 65 | 55 | 75 | 20 |
| Ethylhexyl stearate | 5 | | 5 | 5 | 5 | 5 |

Stability studies have been performed and the stability of compositions above is confirmed up to 36 months.

**Example 2 - Formulations with other hydrocarbons**

The following compositions are prepared in a similar manner to the ones in example 1. The compounds are blended in a recipient at room temperature (22°C ± 2°C) with a continuous mixing during about 20 minutes at low to medium speed to prevent incorporation of air. The product can be stored in an enclosed cover stainless steel storage tank at controlled room temperatures of 15-30°C until packaging.

| Ingredients | Compositions (w/w %) | | | | |
|---|---|---|---|---|---|
| | Ex 2.1 | Ex 2.2 | Ex 2.3 | Ex 2.4 | Ex 2.5 |
| Paraffin | 50 | | | | |
| Petroleum jelly | | 50 | | | |
| Isohexadecane | | | 50 | | |
| Isoeicosane | | | | 50 | |
| Squalene | | | | | 50 |
| Decamethylcyclopentasiloxane | 45 | 45 | 45 | 45 | 45 |
| Ethylhexyl stearate | 5 | 5 | 5 | 5 | 5 |

**Example 3 - Formulations with Tea Tree (Melaieuca alternifolia) oil**

The following compositions were prepared in a similar manner to the ones in example 1. The compounds are blended in a recipient at room temperature (22°C ± 2°C) with a continuous mixing during about 20 minutes at low to medium speed to prevent incorporation of air. The product can be stored in an enclosed cover stainless steel storage tank at controlled room temperatures 15-30°C until packaging.

| Ingredients | Compositions (w/w %) | | |
|---|---|---|---|
| | Ex 3.1 | Ex 3.2 | Ex 3.3 |
| Squalane | 99 | 50 | |
| Isopropyl myristate | | | 50 |
| Cyclomethicone | | 45 | 45 |
| Ethylhexyl stearate | | 4 | 4 |
| Tea Tree oil | 1 | 1 | 1 |

**Example 4** - **Comparative examples** with **some pediculicide compositions in the market.**

| Ingredients | Compositions (w/w %) | | |
|---|---|---|---|
| | Ex 4.1 | Ex 4.2 | Ex 4.3 |
| Isopropyl myristate | 50 | | |
| Cyclomethicone | 50 | | |
| Permethrin | | 1.5 | |
| Decamethylcyclopentasiloxane | | | 5.0 |
| Octamethyltrisiloxane | | | 4.0 |
| Other non-active excipients | | 98.5 | 91 |

**Example 5 - Procedure for evaluating ectoparasiticidal activity - efficacy against head lice crawling stages (late nymphs and adults)**

Biological test species: *Pediculus humanus capitis* De Geer, 1778 (Head lice).

Stage and number: groups of 10 specimens; adults and nymphs of 3rd stage (males and females).

Head lice were collected from the heads of volunteers. Crawling stages were obtained by mechanical action of a nit comb and immediately settled in Petri dishes and incubated at 25°C and 60% RH until the time of testing, between three and five hours after collection.

Product samples were conditioned at the same environmental conditions as treatment application, at $25 \pm 1$ °C and $60 \pm 10$ % RH. Once treatments were applied, lice were incubated in the dark at the same environmental conditions.

Treatments were conducted in 10 mL of product poured in 5 cm diameter Petri dishes. For negative controls, tap water was used instead and tests were prepared in the same way.

Crawling stages were transferred with great care and soft tweezers to the center of a Petri dish lined with moistened Whatman n° 1 filter paper with 0.1 mL of tap water. Only those specimens that moved from the center to the edge of the dish were used, being discarded those that remained motionless.

Lice treatment was conducted by complete immersion method for 5 minutes or 2 minutes by submerging each group of specimens into the product. Then, lice were transferred to a permeable well, washed with tap water for one minute with a wash bottle and settled in 5 cm Petri dishes previously labelled and lined with moistened filter paper Whatman n° 1 with 0.1 mL of tap water, before being introduced in the incubator until evaluation.

Three replicates were conducted for the treatments and three replicates with tap water were used as negative controls.

Crawling stages were observed under the binocular microscope at 5, 15, 30, 60, 180 minutes and 18 and 24 hours post-treatment, with a stereoscopic microscope Olympus SZ61TR with zoom up to 4,5x20 magnifying. Mortality criteria with a unique observer were:

i) Alive: Head lice can walk, grab a hair and move forward.

ii) Vital signs apparent (VSA): Head lice seem to be apparently well but their movements are not coordinated; they can't walk nor grab a hair and move forward (moribund or knocked down).

iii) Vital signs reduced (VSR): Head lice show only slight movements on antennae, legs or digestive tract (moribund or knocked down).

iv) Dead (D): Complete movement cessation (including digestive tract).

Mortality percentage: For data analysis, the number of dead head lice is the result of the combination of knocked down (VSA and VSR) and dead (D). The percentage mortality is calculated in each replicate considering the number of alive specimens and affected specimens (VSA+VSR+D) following the relationship: *N° of affected specimens/ Total n° of specimens x 100*

**Example 6 - Effect of compositions on ectoparasites: efficacy against head lice crawling stages (late nymphs and adults)**

Example 6.1 (t=5 min immersion)

The results are summarized in Table 1.

Table 1: Mean mortality percentage and standard deviation (SD) of head lice crawling stages for composition of Example 1.1, in the control and treatment, at each recording points with 5 minutes immersion.

| Time post-application | % Mortality (Mean $\pm$ SD) | |
|---|---|---|
| | Control | Ex 1.1 |
| 5 min | 0 $\pm$ 0 | 100 $\pm$ 0 |
| 15 min | 0 $\pm$ 0 | 100 $\pm$ 0 |
| 30 min | 0 $\pm$ 0 | 100 $\pm$ 0 |
| 60 min | 0 $\pm$ 0 | 100 $\pm$ 0 |
| 180 min | 0 $\pm$ 0 | 100 $\pm$ 0 |
| 18 hours | 30 $\pm$ 10 | 100 $\pm$ 0 |
| 24 hours | 57 $\pm$ 12 | 100 $\pm$ 0 |

Table 2: Mean mortality percentage and standard deviation (SD) of head lice crawling stages of Examples 1.2, 3.1, 3.2 and 3.3, in the control and treatment, at each recording points with 5 minutes immersion.

| Time post-application | % Mortality (Mean $\pm$ SD) | | | | | |
|---|---|---|---|---|---|---|
| | Control[1] | Ex 1.2 | Ex 3.1 | Ex 3.2 | Control[2] | Ex 3.3 |
| 5 min | 0 $\pm$ 0 | 100 $\pm$ 0 | 100 $\pm$ 0 | 100 $\pm$ 0 | | |
| 15 min | 0 $\pm$ 0 | 100 $\pm$ 0 | 100 $\pm$ 0 | 100 $\pm$ 0 | 0 $\pm$ 0 | 100 $\pm$ 0 |
| 30 min | 0 $\pm$ 0 | 100 $\pm$ 0 | 97 $\pm$ 6 | 100 $\pm$ 0 | 0 $\pm$ 0 | 100 $\pm$ 0 |
| 60 min | 0 $\pm$ 0 | 100 $\pm$ 0 | 97 $\pm$ 6 | 100 $\pm$ 0 | 0 $\pm$ 0 | 100 $\pm$ 0 |
| 180 min | 0 $\pm$ 0 | 100 $\pm$ 0 | 97 $\pm$ 6 | 100 $\pm$ 0 | 0 $\pm$ 0 | 100 $\pm$ 0 |
| 18 hours | 20 $\pm$ 10 | 100 $\pm$ 0 | 100 $\pm$ 0 | 100 $\pm$ 0 | 23 $\pm$ 6 | 100 $\pm$ 0 |
| 24 hours | 50 $\pm$ 10 | 100 $\pm$ 0 | 100 $\pm$ 0 | 100 $\pm$ 0 | | |
| (1) Control results corresponding to Ex 1.2, 3.1 and 3.2 | | | | | | |
| (2) Control results corresponding to the Ex 3.3 | | | | | | |

Crawling stages efficacy in all the trials is 100% after five minutes of immersion at five minutes post-treatment application. Due to 100% mortality in the treatments at 18 and 24 hours, Abbot's correction does not apply.

Example 6.2 (t=2 min immersion)

The results obtained after 2 minute immersion are summarized in Table 3.

Table 3: Mean mortality percentage and standard deviation (SD) of head lice crawling stages, in the control and treatment, at each recording points with 2 minute immersion.

| Time post-application | % Mortality (Mean $\pm$ SD) | | |
|---|---|---|---|
| | Control | Ex 1.1 | Ex 1.3 |
| 5 min | 0 $\pm$ 0 | 100 $\pm$ 0 | 100 $\pm$ 0 |

(continued)

| Time post-application | % Mortality (Mean ± SD) | | |
|---|---|---|---|
| | Control | Ex 1.1 | Ex 1.3 |
| 15 min | 0 ± 0 | 100 ± 0 | 100 ± 0 |
| 30 min | 0 ± 0 | 100 ± 0 | 100 ± 0 |
| 60 min | 0 ± 0 | 100 ± 0 | 100 ± 0 |
| 180 min | 0 ± 0 | 100 ± 0 | 100 ± 0 |
| 18 hours | 45 ± 10 | 100 ± 0 | 100 ± 0 |

Crawling stages efficacy is 100% after 2 minutes of immersion at five minutes post-treatment application. Due to 100% mortality at 18 hours in the treatments, Abbot's correction does not apply.

Example 6.3 - Comparatives formulas (t=5 min immersion)

Results observed after at different time post-application and obtained after 5 minute immersion of the formulas in the market are summarized in Table 4.

Table 4: Mean mortality percentage and standard deviation (SD) of head lice crawling stages for comparatives compositions of Example 4.2 and Example 4.3, in the control and treatment, at each recording points with 5 minutes immersion.

| Time post-application | % Mortality (Mean ± SD) | | |
|---|---|---|---|
| | Control | Ex 4.2 | Ex 4.3 |
| 60 min | 0 ± 0 | 17 ± 21 | 10 ± 10 |
| 180 min | 0 ± 0 | 13 ± 15 | 7 ± 12 |
| 6 hours | 0 ± 0 | 20 ± 10 | 17 ± 21 |
| 8 hours | 0 ± 0 | 20 ± 10 | 17 ± 21 |
| 18 hours | 0 ± 0 | 27 ± 6 | 27 ± 23 |
| 24 hours | 30 ± 10 | 47 ± 6 | 60 ± 20 |

The comparative compositions of examples 4.2 and 4.3 shown a mean pediculicide mortality of 27% at 18 hours post-treatment application. Even after 24 hours post-treatment application the mortality is of 47% and 60%, respectively. The efficacy of the formulas in the market is clearly much lower than the ones of the present invention.

**Example 7 - Procedure for Evaluating Compositions for ectoparasiticidal activity - efficacy against head lice ova**

Biological test species: *Pediculus humanus capitis* De Geer, 1778 (Head lice).

Stage and number: groups of 10 ova.

Head lice ova were collected from volunteers, with Ethic Committee approval, by specialized staff by cutting the hair with round scissors, considering only those eggs viable (grey in colour) and located less than 1 cm from the scalp. Once obtained, the hair shafts with the eggs were settled in Petri dishes and incubated at 25°C and 60±10% relative humidity until the beginning of the assay, between twelve and fifteen hours after collection.
Product samples were conditioned at the same environmental conditions as treatment application, at 25±1°C and 60±10% RH. Once treatments were applied, the eggs were incubated in the dark at 29±1°C y 70±5% HR.
Treatments were conducted in 10 mL of product poured in 5 cm diameter Petri dishes. Negative controls, tap water, were prepared in the same way.
Ova were inspected, under a stereoscopic microscope Olympus SZ61TR with zoom up to 4,5x20 magnifying, to determine the viability and development stage. Only those that showed to be in good shape, with an embryo and the surface and operculum intact, were selected. Then, they were classified in different development stages on behalf of their morphological characters as early (no differentiation) or late (red or black eye spot and appendages and/or embryonic movements). Once the number of viable ova available in each of the development stages was determined, these were distributed evenly in groups among replicates.

Ova treatment was conducted by complete immersion method for 5 minutes by submerging each group of hair shafts (3 cm) into the product. Then, the hair shafts were transferred to a permeable well and washed with tap water for one minute with a wash bottle and settled in 5 cm Petri dishes previously labelled and lined with moistened filter paper Whatman n° 1 with 0.1 mL of tap water, before being introduced in the incubator until evaluation.

Three replicates were conducted for the treatments and three replicates with tap water were used as negative controls.

Observations on the development of head lice eggs were made at 7 and 14 days after treatment, under a stereoscopic microscope Olympus SZ61TR with zoom up to 4.5x20 magnifying.

Mortality criteria

Criteria used for data recording were:

i) Complete Hatching (CH): Vital lice hatch.
ii) Incomplete Hatching (IH): The operculum is opened but the louse is entirely or partially inside the egg.
iii) No hatching: With operculum closed, the egg is intact or shrunk. Development stage is stated as early stage (ES) or late stage (LS).

Mortality percentage

For data analysis, the number of dead ova is the result of the combination of incomplete hatching (IH) and not hatched, either in ES or LS. The percentage of mortality is calculated in each replicate considering the number of hatched ova and dead ova (ii,iii) following the relationship:

$$Number\ of\ dead\ ova\ /Total\ number\ of\ ova\ x\ 100.$$

The final value is obtained after correction with mortality in the controls using Abbot's formula:

$$Abbot\ correction = \frac{Mortality\ \%\ treatment - Mean\ \%\ mortality\ controls}{100 - Mean\ \%\ mortality\ controls}\ x\ 100$$

**Example 8 - Effect of compositions on ectoparasites: Efficacy against head lice ova**

Example 8.1 - t=5 minutes immersion

The results are summarized in Table 5.

Table 5: Ova mortality percentages obtained in each replicate of the treatments with the composition of Example 1.1, along with the mean percentage and standard deviations (SD) and the final percentage considering Abbot's correction.

| Replicate | % Mortality (Mean ± SD) | | % Mortality after Abbott's correction |
|---|---|---|---|
| | Control | Example 1.1 | |
| R-1 | 20 | 100 | 100 |
| R-2 | 0 | 70 | 67.86 |
| R-3 | 0 | 90 | 89.29 |
| Mean ± SD | 6.67 ± 11.55 | 86.67 ± 15.28 | 85.71 ± 16,37 |

The treatment group shows a mean ovicidal mortality of 87% at 14 days post-treatment application. Applying Abbot's correction, considering mortality in the controls, the final value is 86%.

Example 8.2 - t=5 minutes immersion

In relation to the composition of Example 3.3, the results are summarized in Table 6.

Table 6: Ova mortality percentages obtained after 5 of immersion, along with the mean percentage and standard deviations (SD) and the final percentage considering Abbot's correction

| Time of | % Mortality (Mean ± SD) | | % Mortality after Abbott's |
|---|---|---|---|
| immersion | Control | Example 3.3 | correction |
| 5 minutes | 6.7 ± 5,8 | 60.0 ± 10,0 | 53.4 ± 10,0 |

The treatment group shows a mean ovicidal mortality of 60% with 5 minutes immersion at 14 days post-treatment application. Applying Abbot's correction, considering mortality in the controls, the final value is 53.4%.

Example 8.3 - t=2 minutes immersion

The results are summarized in Table 7.

Table 7: Ova mortality percentages obtained in each replicate of the treatments with the composition of Example 1.1 and Example 1.3 after 2 min of immersion of the product.

| Time of immersion | % Mortality (Mean ± SD) | | | |
|---|---|---|---|---|
| | Example 1.1 | | Example 1.3 | |
| | | after Abbott's correction | | after Abbott's correction |
| 2 minutes | 43.33 ± 15.05 | 26.98 ± 24.96 | 53.33 ± 19.44 | 47.11 ± 20.48 |

The treatment with composition of Example 1.1 and 1.3 show a mean ovicidal mortality of 43% and 53%, respectively, at 14 days post-treatment application. Applying Abbot's correction, considering mortality in the controls, the final value is 27% and 47%, respectively.

Example 8.4 - Comparative formula

In relation to the compositions of comparative Example 4, the results are summarized in Table 8.

Table 8: Ova mortality percentages obtained after 5 minutes of immersion, along with the mean percentage and standard deviations (SD) and the final percentage considering Abbot's correction.

| Time of immersion | % Mortality (Mean ± SD) | | | | | | |
|---|---|---|---|---|---|---|---|
| | Control | Example 4.1 | | Example 4.2 | | Example 4.3 | |
| | | | after Abbott's correction | | after Abbott's correction | | after Abbott's correction |
| 5 minutes | 6.7 ± 5.8 | 13.3 ± 5.8 | 7.07 | 6.7 ± 11.5 | 0 | 3.3 ± 5.8 | 0 |

The composition of example 4.1 shows a mean ovicidal mortality of 13% at 14 days post-treatment application Applying Abbot's correction, considering mortality in the controls, the final value is 7%. The other two formulas in the market after applying Abbot's correction did not show any ovicidal effect.
The efficacy of the formulas in the market is much lower than the ones of the present invention.

**Example 9 - Clinical study on the sensitizing potential of the formulation in healthy adult volunteers according to Marzulli-Maibach method: human repeated insult patch test**

The main objective of this study was to verify irritation and sensitizing potential of the formulations of Example 1 after repeated applications under occlusion, 3 times a week during 3 consecutive weeks (Induction Phase), on the back of 50 healthy adult volunteers with different skin types (I to IV), followed by a 2-weeks rest period and a single application of the product under patch to a well delimited site and to the induction site (Challenge Phase).
The study showed no irritating potential during the Induction Phase of the study and no sensitizing potential during the Induction Phase.
Under the experimental study conditions, it is considered that the formulations of Example 1 are non-irritating and non-sensitizing.

**Example 10 - Clinical study to assess the skin tolerance under normal conditions of use in children from 1 year of age under dermatological and paediatric control: Use test**

The main objective of this study was to verify the absence of cumulative reactions and cumulative irritations (functional and physical signs) associated with the application of the formulations of Example 1 under normal conditions of use for 2 weeks in 20 healthy children of both sexes, aged between 1 and 3 years. This study was conducted under dermatological control (subjects individually examined by a Dermatologist Investigator) and paediatric (subjects individually examined by a Paediatric Investigator).
The experimental phase of this trial lasted for 14 days, in which the parents / guardians of the children made 3 visits: day 0, day 7 and day 14.
On days 0 and 7, the lotion was applied to the dry hair of the children by performing a gentle massage so that hair and scalp were well impregnated, insisting on the area behind the ears and the nape. The following amounts of the product were used depending on the type of hair:

- Short or very fine hair: 1/4 bottle
- Half mane: 1/2 boat
- Long or very thick hair: 1 bottle (100 ml)

After applying the product, the product was left for 5 minutes and then the hair was washed with the usual shampoo, rinsing with plenty of water. The hair was dried hair in the usual way.
In the 3 visits, the researchers evaluated the area of application of the product after its use to assess possible adverse reactions or irritation produced by the products. The products did not produce any undesirable skin reactions in the study participants after 14 days of use, according to the study's researchers. During the 14-day study period, none of the parents / guardians reported any undesirable reactions.
Under the experimental study conditions, it is considered that the formulations of Example 1 are very well tolerated at skin level and it can be stated that the product has been "tested under dermatological and paediatric control".

**Claims**

1. Squalane for use as a medicament.

2. The product according to claim 1 for use in killing ectoparasites and/or their ova on a subject.

3. A composition for use in killing ectoparasites and/or their ova on a subject comprising squalane, wherein squalane is present in an amount of at least 5 % by weight in respect of the total amount of the composition.

4. The composition for use according to the preceding claim, wherein the composition does not comprise Lippia javanica essential oil, preferably the total amount of squalane present in the composition ranges from 5 to 20 % by weight in respect of the total amount of the composition.

5. The composition for use according to claim 3, wherein squalane is present in an amount of more than 20 % by weight in respect of the total amount of the composition.

6. The composition for use according to any one of claims 3 to 5, wherein the composition further comprises at least a cyclic siloxane, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane.

7. The composition for use according to any one of claims 3 to 6, wherein the composition comprises at least a further emollient, preferably the further emollient is a fatty acid ester, more preferably selected from cetyl palmitate, decyl oleate, ethyl oleate, isopropyl myristate, isopropyl palmitate, ethylhexyl stearate and mixtures thereof, even more preferably the composition comprises ethylhexyl stearate.

8. The composition for use according to any one of claims 3 to 7, wherein at least 75% of ectoparasites present are killed within 4 hours after a 10 minute exposure to the composition, preferably wherein at least 75% of ectoparasites present are killed within 1 hours after a 10 minute exposure to the composition, more preferably wherein at least 75% of ectoparasites present are killed within 10 minutes after a 5 minute exposure to the composition, even more preferably wherein at least 80% of ectoparasites present are killed within 5 min after a 5 minute exposure to the composition.

9. The composition for use according to any one of claims 3 to 8, wherein at least 60% of the ova are killed within 14 days after 10 minute exposure, preferably wherein at least 80% of the ova are killed within 14 days after 10 minute exposure, more preferably wherein at least 80% of the ova are killed within 14 days after 5 minute exposure.

10. A topical composition comprising squalane and at least a siloxane.

11. The composition according to claim 10, wherein squalane is present in an amount of at least 30% by weight in respect of the total amount of the composition, preferably the cyclic siloxane is selected from cyclomethicone, hexamethylcyclotrisiloxane, octamethylcyclotetrasiloxane, dodecamethylcyclohexasiloxane, decamethylcyclopentasiloxane, hexamethyldisiloxane and mixtures thereof, more preferably the composition comprises decamethylcyclopentasiloxane.

12. The composition according to any one of claims 10 to 11, wherein the composition comprises at least a further emollient, preferably the further emollient is a fatty acid ester, more preferably selected from cetyl palmitate, decyl oleate, ethyl oleate, isopropyl myristate, isopropyl palmitate, ethylhexyl stearate and mixtures thereof, even more preferably the composition comprises ethylhexyl stearate.

13. A method of killing ectoparasites and/or their ova on a subject comprising, topically administering to an area on the subject where ectoparasites or their ova are present squalane or a composition as defined in any one of the claims 3 to 12.

14. Use of a squalane or a composition as defined in any one of the claims 3 to 12 for the manufacture of a medicament for killing ectoparasites and/or their ova on a subject.

15. A kit for use in treating ectoparasite infestations comprising squalane or a composition as defined in any one of claims 3 to 12 in a package or other enclosure; and instructions describing how to use the items included in the kit to kill ectoparasites, preferably the kit further comprises a nit comb.

Europäisches
Patentamt

European
Patent Office

Office européen
des brevets

**EUROPEAN SEARCH REPORT**

Application Number

EP 17 38 2439

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/160029 A1 (ASMUS ROBERT A [US] ET AL) 31 October 2002 (2002-10-31) <br> * pages 18-19; example 15 * <br> * page 22; example 24A * | 3-5,7-10 | INV. <br> A01N27/00 <br> A01P7/00 <br> A61K31/01 |
| X | TSUKASA KANII ET AL: "Clinical Evaluations of Squalane in Patients with Xerotic Dermatoses and the Results of Patch Test of Squalane and its Moisturizing Effects", <br> SKIN RESEARCH, <br> vol. 33, no. 2, <br> 1 January 1991 (1991-01-01), pages 155-163, XP055403564, <br> DOI: <br> http://doi.org/10.11340/skinresearch1959.33.155 <br> * abstract * <br> * page 162, right-hand column, lines 1-4 * | 1,3,5,8,9 | |
| X | EP 0 226 337 A1 (LILLY CO ELI [US]) 24 June 1987 (1987-06-24) <br> * page 31; example 13 * <br> * claims 4-7, 11, 12,14 * | 3-10,12 | TECHNICAL FIELDS SEARCHED (IPC) <br><br> A01N <br> A61K |
| X | WO 2010/093573 A2 (AMCOL INTERNATIONAL CORP [US]; SENGUPTA ASHOKE K [US]) 19 August 2010 (2010-08-19) <br> * examples I, II, V * | 3-10,12 | |
| A | DAVID C. FLINDERS ET AL: "Pediculosis and Scabies", <br> AMAERICAN FAMILY PHYSICIAN, <br> vol. 69, no. 2, <br> 15 January 2004 (2004-01-15), pages 341-348, XP055403673, <br> * "Treatment of Head Lice"; <br> page 342 - page 344 * | 1-15 | |

-/--

The present search report has been drawn up for all claims

| Place of search | Date of completion of the search | Examiner |
|---|---|---|
| Munich | 5 September 2017 | Davies, Maxwell |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

page 1 of 2

Europäisches
Patentamt

European
Patent Office

Office européen
des brevets

**EUROPEAN SEARCH REPORT**

Application Number

EP 17 38 2439

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2008/101131 A1 (NONITS LLC [US]; DE WOLFF RICKI [US]) 21 August 2008 (2008-08-21) * claim 27 * | 1-15 | |
| A | WO 2013/011501 A1 (PERRIGO ISRAEL PHARMACEUTICALS LTD [IL]; ARNON MICHAEL N [IL]; KAMENET) 24 January 2013 (2013-01-24) * page 13, paragraph [0063] * | 1-15 | |

TECHNICAL FIELDS
SEARCHED      (IPC)

The present search report has been drawn up for all claims

| Place of search | Date of completion of the search | Examiner |
|---|---|---|
| Munich | 5 September 2017 | Davies, Maxwell |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 38 2439

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2017

| Patent document cited in search report | Publication date | Patent family member(s) | Publication date |
|---|---|---|---|
| US 2002160029 A1 | 31-10-2002 | US 2002160029 A1<br>US 2004071748 A1<br>US 2006121071 A1<br>US 2010331422 A1 | 31-10-2002<br>15-04-2004<br>08-06-2006<br>30-12-2010 |
| EP 0226337 A1 | 24-06-1987 | AR 240245 A1<br>AU 591725 B2<br>BR 8605632 A<br>DE 3685003 D1<br>DK 545086 A<br>EP 0226337 A1<br>IE 59661 B1<br>JP S62120309 A<br>NZ 218285 A<br>PH 23053 A<br>US 4797272 A<br>US 4797273 A<br>ZA 8608629 B | 30-03-1990<br>14-12-1989<br>18-08-1987<br>27-05-1992<br>16-05-1987<br>24-06-1987<br>09-03-1994<br>01-06-1987<br>27-07-1989<br>27-03-1989<br>10-01-1989<br>10-01-1989<br>27-07-1988 |
| WO 2010093573 A2 | 19-08-2010 | CA 2750198 A1<br>EP 2396085 A2<br>US 2010202985 A1<br>WO 2010093573 A2 | 19-08-2010<br>21-12-2011<br>12-08-2010<br>19-08-2010 |
| WO 2008101131 A1 | 21-08-2008 | CA 2678357 A1<br>EP 2124576 A1<br>US 2008193387 A1<br>WO 2008101131 A1 | 21-08-2008<br>02-12-2009<br>14-08-2008<br>21-08-2008 |
| WO 2013011501 A1 | 24-01-2013 | CA 2842277 A1<br>EP 2734189 A1<br>US 2014219928 A1<br>WO 2013011501 A1 | 24-01-2013<br>28-05-2014<br>07-08-2014<br>24-01-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4147800 A
- US 6303581 B
- GB 2204243 B2
- WO 0001347 A
- WO 2001019190 A
- WO 2001040446 A
- WO 03092583 A
- US 20130018016 A1

### Non-patent literature cited in the description

- **J. ROSE.** The Aromatherapy Book - Applications & Inhalations. North Atlantic Books, 1992